# EUROPEAN PATENT APPLICATION

(11) **EP 3 808 856 A1**
(43) Date of publication of application: **21.04.2021**
(21) Application number: 19819583.6
(22) Date of filing: 14.06.2019
(51) Int. Cl.: C12Q 1/04, C12Q 1/6834, C12Q 1/6837, C12Q 1/6841, C12Q 1/686, C12Q 1/6869, C12Q 1/6881, C12Q 1/6897, G01N 33/50, G01N 33/53, G01N 33/68, C12N 15/09

(54) **UNDIFFERENTIATED CELL DETECTION METHOD**

(30) Priority: 15.06.2018 JP 2018115025
(71) Applicant: Public University Corporation Yokohama City University, Yokohama-shi, Kanagawa 236-0027 (JP)
(72) Inventor: TANIGUCHI, Hideki, Yokohama-shi, Kanagawa 236-0004 (JP); SEKINE, Keisuke, Yokohama-shi, Kanagawa 236-0004 (JP)
(74) Representative: J A Kemp LLP
(86) International application number: PCT/JP2019/023599
(87) International publication number: WO 2019/240247

(57) **Abstract**

The present invention provides a marker gene capable of detecting the undifferentiated cells that remain or become included in a differentiated cell population. Undifferentiated cells present in a differentiated cell population are detected by using at least one gene selected from the group consisting of *ESRG*, *VSNL1*, *THY1*, *SFRP2*, *SPP1*, *USP44* and *CNMD* as an undifferentiation marker. A method of detecting undifferentiated cells; a method of using the gene as an undifferentiation marker; and a kit for detecting undifferentiated cells. A method of selecting an undifferentiated cell clone is also provided.

## Description

### TECHNICAL FIELD

The present invention relates to a method for detecting undifferentiated cells.

### BACKGROUND ART

From the viewpoint of the risk of oncogenesis, it is extremely important to detect and evaluate how much of undifferentiated iPS cells remain or become included in differentiated cell populations for use in regenerative medicine. To date, the following methods have been reported as techniques for detecting and evaluating such remaining or inclusion of undifferentiated iPS cells. Briefly, a method in which iPS cell specific genes are detected by quantitative PCR (qPCR) (Non-Patent Document No.1) and a method in which differentiated cells are re-cultured under undifferentiated cell maintenance conditions have been reported (Non-Patent Document No. 2).

Among the conventional methods, the re-culturing method has an advantage of high accuracy since colonies are formed from undifferentiated iPS cells that have become included in differentiated cells, but it takes more than one week for detection. Therefore, the method using quantitative PCR is superior in that it can be implemented in a simple and quick manner.

However, with respect to *LIN28* reported previously (Non-Patent Document No. 1), while its expression is low in a differentiated cell of organ/tissue (retinal pigment epithelial cell), expression is observed in other differentiated cells (such as hepatic cells) and cells that result from directed differentiation of iPS cells. Therefore, *LIN28* cannot be used for detecting the remaining or inclusion of undifferentiated iPS cells in a wide range of differentiated cells.

### PRIOR ART LITERATURE

### Non-Patent Documents

Non-Patent Document No. 1: PLoS One. 2014 27;9(10):e110496
Non-Patent Document No. 2: PLoS One. 2012;7(5):e37342

### DISCLOSURE OF THE INVENTION

### PROBLEM FOR SOLUTION BY THE INVENTION

It is an object of the present invention to provide a marker gene by which even undifferentiated cells that remain or become included in differentiated cells other than retinal pigment epithelial cell can be detected.

### MEANS TO SOLVE THE PROBLEM

The present inventors have identified marker genes that are applicable to a wide variety of differentiated cells and which are capable of universal detection of the remaining or inclusion of undifferentiated iPS cells.

No marker gene is ideal if it satisfies only the following two requirements:
1. It is specifically expressed in undifferentiated iPS cells;
2. Its expression is extremely low in cell lineages other than undifferentiated iPS cells. An additional criterion to be met is the following requirement that must be satisfied to enable detection even when only a few undifferentiated cells are present in differentiated cells:
3. Its expression is extremely high in undifferentiated iPS cells.

Then, the present inventors have found, as genes that satisfy the above criteria, *ESRG*, *SFRP2*, *VSNL1*, *THY1*, *SPP1*, *USP44* and *CNMD* (*LECT1*) which are expressed highly in undifferentiated iPS cells and whose expression becomes 0.1% or less in differentiated endodermal cells. By using these genes, it has become possible to detect the remaining or inclusion of undifferentiated iPS cells to as low as 0.005% in differentiated cells of organs/tissues that defied evaluation by conventional indicators. Further, since the expression of these marker genes also becomes 0.1% or less even in cells that have differentiated to mesodermal and ectodermal cells, it is believed that these genes are markers capable of detecting the remaining or inclusion of undifferentiated iPS cells in any one of differentiated endodermal, mesodermal or ectodermal cells.

A summary of the present invention is as described below.
(1) A method of detecting undifferentiated cells, comprising measuring the expression level and/or the promoter activity of at least one gene selected from the group consisting of *ESRG*, *VSNL1*, *THY1*, *SFRP2*, *SPP1*, *USP44* and *CNMD* in a differentiated cell population.
(2) The method of (1) above, wherein the undifferentiated cell is embryonal carcinoma cell (EC cell), embryonic stem cell (ES cell), induced pluripotent stem cell (iPS cell) or embryonic germ cell (EG cell), and the differentiated cell population is a population of cells which have been differentiated from the undifferentiated cell.
(3) The method of (1) or (2) above, wherein the differentiated cell population is a population of differentiated endodermal, mesodermal or ectodermal cells.
(4) The method of (3) above, wherein the differentiated endodermal cells are hepatic endoderm cells.
(5) The method of (3) above, wherein the differentiated mesodermal cells are *septum transversum* mesenchyme cells, mesenchymal cells or vascular endothelial cells.
(6) The method of (3) above, wherein the differentiated ectodermal cells are neural stem cells, neural crest cells or neural cells.
(7) The method of any one of (1) to (6) above, wherein the expression level of the gene is measured as the amount of mRNA or protein.
(8) The method of any one of (1) to (7) above, wherein the expression level of the gene is measured by qPCR, digital PCR, immunostaining, *in situ* hybridization, RNA sequencing, microarray, NanoString, antibody array, flow cytometry or mass spectrometry.
(9) A method of selecting a highly safe undifferentiated cell clone, comprising measuring the expression level and/or the promoter activity of at least one gene selected from the group consisting of *ESRG*, *VSNL1*, *THY1*, *SFRP2*, *SPP1*, *USP44* and *CNMD* in differentiated endodermal, mesodermal or ectodermal cells that result from directed differentiation of an undifferentiated cell clone.
(10) The method of (9) above, wherein the undifferentiated cell clone is an embryonal carcinoma cell (EC cell) clone, an embryonic stem cell (ES cell) clone, an induced pluripotent stem cell (iPS cell or iPSC) clone or an embryonic germ cell (EG cell) clone.
(11) The method of (10) above, wherein the undifferentiated cell clone is an iPS cell clone.
(12) A method of detecting undifferentiated cells, comprising measuring the expression level and/or the promoter activity of at least one gene selected from the group consisting of *ESRG*, *VSNL1*, *THY1*, *SFRP2*, *SPP1*, *USP44* and *CNMD* in a tissue formed by transplanting differentiated cells into a model animal.
(13) A method of using at least one gene selected from the group consisting of *ESRG*, *VSNL1*, *THY1*, *SFRP2*, *SPP1*, *USP44* and *CNMD* as an undifferentiation marker for detecting undifferentiated cells in a differentiated cell population.
(14) A kit for detecting undifferentiated cells, comprising a reagent capable of detecting the expression of at least one gene selected from the group consisting of *ESRG*, *VSNL1*, *THY1*, *SFRP2*, *SPP1*, *USP44* and *CNMD* and/or a reagent capable of measuring the promoter activity of the gene.
(15) The kit of (14) above, wherein the reagent capable of detecting the expression of the gene is primers, probes or antibodies.
(16) The kit of (14) above, wherein the reagent capable of measuring the promoter activity of the gene is a gene sequence in which a reporter protein is ligated downstream of the promoter or a vector incorporating the gene sequence.

### EFFECT OF THE INVENTION

According to the present invention, it is possible to detect and evaluate how much of undifferentiated cells remain or become included in a differentiated cell population and this contributes to reducing the risk of oncogenesis in differentiated cells for use in regenerative medicine.

The present specification encompasses the contents disclosed in the specification and/or drawings of Japanese Patent Application No. 2018-115025 based on which the present patent application claims priority.

### BRIEF DESCRIPTION OF THE DRAWINGS

[Fig. 1] Mouse *LIN28A* expression in hepatic cells during liver development (microarray data). Compared to adult (8w), expression is high until E13.5.
[Fig. 2] *LIN28A* expression at respective differentiation stages of directed differentiation from human iPSCs to hepatic cells. *LIN28A* expression is also high in definitive endoderm (DE) and hepatic endoderm (HE).
[Fig. 3] qPCR of genes extracted by microarray. Upon confirmation of expression by qPCR, genes whose expression is especially low in hepatic cells were extracted.
[Fig. 4] Method of detecting undifferentiated iPSC by culturing. Hepatic endoderm cells (HE) resulting from directed differentiation of iPSC were cultured under undifferentiated iPSC culture conditions. Then, colonies formed were immunostained to identify undifferentiated iPSC. The method disclosed in Tano K et al., PloS One. 2014; 9(10):e110496 was optimized for this experiment.
[Fig. 5] Method of detecting undifferentiated iPSC by culturing. Hepatic endoderm cells (HE) resulting from directed differentiation of iPSC were cultured under undifferentiated iPSC culture conditions. Then, colonies formed were immunostained to identify undifferentiated iPSC. The method disclosed in Tano K et al., PloS One. 2014; 9(10):e110496 was optimized for this experiment.
[Fig. 6] Correlation between the number of undifferentiated iPS cells detected by the technique of Figs. 4 and 5 (Y axis) and marker gene expression (X axis).
[Fig. 7] Examination of detection sensitivity of marker genes by undifferentiated iPSC spike-in experiments. Undifferentiated iPSCs were mixed into hepatic endoderm cells. The resultant experiment groups were compared to control group without spiking of undifferentiated iPSCs, to thereby examine detection sensitivity by qPCR. *p<0.05.
[Fig. 8] Expression of undifferentiation markers and evaluation of residual iPSC in mesodermal cells (Mesoderm) and mesoderm-derived cells (mesenchymal cells). Differentiation to mesenchymal cells was confirmed by expression of *ALCAM*, *CD73*, *PDGFRB* and *DES.* Expression of undifferentiation detection marker genes and detection of undifferentiated iPSC by the technique of Figs. 4 and 5 (n=3; detection of undifferentiated cell was zero).
[Fig. 9] Expression of undifferentiation markers and evaluation of residual iPSC in mesodermal cells (Mesoderm) and mesoderm-derived cells (vascular endothelial cells). Differentiation to vascular endothelial cells was confirmed by expression of *CD34*, *PECAM1*, *CDH5* and *KDR.* Expression of undifferentiation detection marker genes and detection of undifferentiated iPSC by the technique of Figs. 4 and 5 (n=3; detection of undifferentiated cell was zero).
[Fig. 10] Expression of undifferentiation markers and evaluation of residual iPSC in ectodermal cells (Ectoderm) and ectoderm-derived cells (neural stem cells (NSC) and neural cells (neuron)). Differentiation to neural stem cells was confirmed by expression of *PAX6, SOX1, NEST* and *TUBB3.* Expression of undifferentiation detection marker genes and detection of undifferentiated iPSC by the technique of Figs. 4 and 5 (n=3; detection of undifferentiated cell was zero).
[Fig. 11] Expression of undifferentiation marker genes in ectodermal cells (Ectoderm) and ectoderm-derived cells (neural crest cells: NCC) was examined by qPCR.
[Fig. 12] iPS cells underwent directed differentiation to individual cells derived from three germ layers using STEMdiff™ Trilineage Differentiation Kit (STEMCELL Technologies). Then, it was confirmed by immunostaining and qPCR that directed differentiation was performed successfully.
[Fig. 13] Expression of marker genes in the cells resulting from directed differentiation in Fig. 12 was examined by qPCR.
[Fig. 14] Residual undifferentiated cell counts in the cells resulting from directed differentiation in Fig. 12 was evaluated by the technique described in Figs. 4 and 5.

### BEST MODES FOR CARRYING OUT THE INVENTION

Hereinbelow, the present invention will be described in detail.

The present invention provides a method of detecting undifferentiated cells, comprising measuring the expression level and/or the promoter activity of at least one gene selected from the group consisting of *ESRG* (HUGO Gene Nomenclature Committee (HGNC) Official Full Name: embryonic stem cell related; NCBI Reference Sequence: NR_027122.1, etc.), *VSNL1* (HGNC Official Full Name: visinin like 1; NCBI RefSeq: NM_003385.4, etc.), *THY1* (HGNC Official Full Name: Thy-1 cell surface antigen; NCBI RefSeq: NM_001311160.1, etc.), *SFRP2* (HGNC Official Full Name: secreted frizzled related protein 2; NCBI RefSeq: NM_003013.2), *SPP1* (HGNC Official Full Name: secreted phosphoprotein 1; NCBI RefSeq: NM_000582.2), *USP44* (HGNC Official Full Name: ubiquitin specific peptidase 44; NCBI RefSeq: NM_001042403.2) and *CNMD* (HGNC Official Full Name: chondromodulin; NCBI RefSeq: NM_001011705.1) in a differentiated cell population.

The undifferentiated cell that is the target of detection may be a cell with pluripotency, e.g., embryonal carcinoma cell (EC cell), embryonic stem cell (ES cell), induced pluripotent stem cell (iPS cell) or embryonic germ cell (EG cell).

Cells constituting the differentiated cell population may be any cells other than undifferentiated cells and, preferably, cells without pluripotency. For example, cells constituting the differentiated cell population are those cells which have been differentiated from an undifferentiated cell that is the target of detection.

The differentiated cell population may be a population of any one of endodermal, mesodermal or ectodermal differentiated cells.

When cells constituting the differentiated cell population are endodermal differentiated cells (such as endodermal cells or endoderm-derived cells), preferable marker genes are *ESRG*, *VSNL1*, *THY1*, *SFRP2*, *SPP1*, *USP44* and *CNMD.* When cells constituting the differentiated cell population are mesodermal differentiated cells (such as mesodermal cells or mesoderm-derived cells), preferable marker genes are *ESRG*, *SFRP2* and *CNMD.* When cells constituting the differentiated cell population are ectodermal differentiated cells (such as ectodermal cells or ectoderm-derived cells), preferable marker genes are *ESRG* and *CNMD.*

Examples of endodermal differentiated cells include, but are not limited to, hepatic endoderm cells.

In one Example described later, the present inventors detected iPS cells (undifferentiated cells) that were caused to become included in a population of hepatic endoderm cells (differentiated cells) resulting from directed differentiation of iPS cells. These hepatic endoderm cells are hepatic progenitor cells designated iPSC-HE (hepatic endoderm) at day 10 of directed differentiation treatment from iPS cells to hepatic cells (Nature 499:481-484 (2013); Japanese Patent No. 6124348 "Method of Preparing Tissues and Organs"). According to measurement by qPCR, it is believed that *ESRG*, *VSNL1*, *THY1*, *SFRP2*, *SPP1*, *USP44* and *CNMD* are effective marker genes for detecting iPS cells in hepatic endoderm cell populations.

Examples of mesodermal differentiated cells include, but are not limited to, *septum transversum* mesenchyme cells, mesenchymal cells and vascular endothelial cells.

In one Example described later, the present inventors detected iPS cells (undifferentiated cells) that were caused to become included in a population of mesenchymal cells (differentiated cells) resulting from directed differentiation of iPS cells. These mesenchymal cells are mesenchymal stem/progenitor cells (mesoderm-derived cells) designated iPSC-STM/MC [iPS cell-derived *septum transversum* mesenchyme cells/iPS cell-derived mesenchymal cells (septum transversum mesenchyme/mesenchymal cells)] (Cell Rep. 21:2661-2670, 2017) which received directed differentiation treatment from iPS cells to mesenchymal cells. These mesenchymal cells are CD166 positive and do not express *CD31* (*PECAM1*)*,* a vascular endothelial marker. According to measurement by qPCR, it is believed that *ESRG, SFRP2* and *CNMD* are effective marker genes for detecting iPS cells in mesenchymal cell populations.

Further, in one Example described later, the present inventors detected iPS cells (undifferentiated cells) that were caused to become included in a population of vascular endothelial cells (differentiated cells) resulting from directed differentiation of iPS cells. These vascular endothelial cells are endothelial progenitor cells (mesoderm-derived cells) designated iPSC-EC (iPS cell-derived endothelial cell) (Cell Rep. 21:2661-2670 (2017)) which received directed differentiation treatment from iPS cells to vascular endothelial cells. In these vascular endothelial cells, expression of the proteins of vascular endothelial markers CD31 (PECAM1) and CD144 can be confirmed by immunostaining. In gene expression analyses, high expression of vascular endothelial markers such as *PECAM1*, *CDH5*, *KDR*, *CD34* and the like is observed; compared to iPS cells before directed differentiation, 10- to more than 100-fold higher expression is observed in these vascular endothelial cells. According to measurement by qPCR, it is believed that *ESRG*, *SFRP2* and *CNMD* are effective marker genes for detecting iPS cells in vascular endothelial cell populations.

Examples of ectodermal differentiated cells include, but are not limited to, neural stem cells, neural crest cells and neural cells.

In one Example described later, the present inventors detected iPS cells (undifferentiated cells) that were caused to become included in a population of neural stem cells (differentiated cells) resulting from directed differentiation of iPS cells. These neural stem cells are neural stem cells (ectoderm-derived cells) designated NSC (iPS cell-derived neural stem cell (Neural stem cells)) (Stem Cell Reports. 5:1010-1022. (2015)) which received directed differentiation treatment from iPS cells to neural stem cells. According to measurement by qPCR, it is believed that *ESRG* and *CNMD* are effective marker genes for detecting iPS cells in neural stem cell populations.

Further, in one Example described later, the present inventors detected iPS cells (undifferentiated cells) mixed in a population of neural cells directed differentiated from iPS cells (Imaizumi et al, Stem Cell Reports, 5:1-13, 2015) (ectoderm-derived cells). According to measurement by qPCR, it is believed that *ESRG* and *CNMD* are effective marker genes for detecting iPS cells in neuronal cell populations.

Still further, in one Example described later, the present inventors detected iPS cells (undifferentiated cells) that were caused to become included in a population of neural crest cells resulting from directed differentiation of iPS cells (Menendez L. et al., Proc Natl Acad Sci USA. 108(48):19240-5. 2011) (ectoderm-derived cells), and obtained the same results as described above.

Further, in one Example described later, expression of marker genes was also detected by qPCR in three individual germ layer-derived cells that were obtained by directed differentiation using STEMdiff™ Trilineage Differentiation Kit (STEMCELL Technologies).

In the present invention, differentiated cells and undifferentiated cells may be derived from human or any of non-human animals.

The expression level of a marker gene may be measured as the amount of the mRNA transcribed from the gene or the amount of the protein translated from the mRNA. Specifically, the expression level of the gene may be measured by qPCR, digital PCR, immunostaining, *in situ* hybridization, RNA sequencing, microarray, NanoString, antibody array, flow cytometry, mass spectrometry or the like.

When expression of at least one gene selected from the group consisting of *ESRG*, *VSNL1*, *THY1*, *SFRP2*, *SPP1*, *USP44* and *CNMD* is confirmed, it is possible to judge that undifferentiated cells are present in the differentiated cell population of interest (detection of undifferentiated cells).

In the present specification, the term "detection" means confirmation of presence, but this term also encompasses confirmation of absence.

According to the method of the present invention, it is possible to detect undifferentiated cells in a differentiated cell population at a detection sensitivity of 0.1% or less, e.g., 0.025%, 0.01% or even 0.005% or 0.0025%. Detection sensitivity can be examined by the spike-in experiment described in an Example provided later.

It also becomes possible to select a highly safe undifferentiated cell clone by measuring the expression level and/or the promoter activity of at least one gene selected from the group consisting of *ESRG*, *VSNL1*, *THY1*, *SFRP2*, *SPP1*, *USP44* and *CNMD* in differentiated endodermal, mesodermal or ectodermal cells that result from directed differentiation of an undifferentiated cell clone. Therefore, the present invention provides a method of selecting a highly safe undifferentiated cell clone in which undifferentiated cells are difficult to remain after directed differentiation, comprising measuring the expression level and/or the promoter activity of at least one gene selected from the group consisting of *ESRG*, *VSNL1*, *THY1*, *SFRP2*, *SPP1*, *USP44* and *CNMD* in any one of differentiated endodermal, mesodermal or ectodermal cells that result from directed differentiation of an undifferentiated cell clone.

The undifferentiated cell clone that is the target of selection may be a cell clone with pluripotency. For example, the undifferentiated cell clone may be embryonal carcinoma cell (EC cell) clone, embryonic stem cell (ES cell) clone, induced pluripotent stem cell (iPS cell or iPSC) clone or embryonic germ cell (EG cell) clone. Among them, iPS cell clone is preferable.

Further, the expression level and/or the promoter activity of at least one gene selected from the group consisting of *ESRG*, *VSNL1*, *THY1*, *SFRP2*, *SPP1*, *USP44* and *CNMD* in a tissue formed by transplanting differentiated cells into a model animal may be measured to detect undifferentiated cells in the tissue. Therefore, the present invention also provides a method of detecting undifferentiated cells, comprising measuring the expression level and/or the promoter activity of at least one gene selected from the group consisting of *ESRG*, *VSNL1*, *THY1*, *SFRP2*, *SPP1*, *USP44* and *CNMD* in a tissue formed by transplanting differentiated cells into a model animal. Suitably, the tissue may be one formed by transplanting differentiated cells into a model animal over a long period of time (e.g., 4 to 54 weeks, preferably 8 to 24 weeks)

According to the present invention, it has become clear that *ESRG*, *VSNL1*, *THY1*, *SFRP2*, *SPP1*, *USP44* and *CNMD* can be used as marker genes for detecting undifferentiated cells present in a differentiated cell population. Therefore, the present invention provides a method of using at least one gene selected from the group consisting of *ESRG*, *VSNL1*, *THY1*, *SFRP2*, *SPP1*, *USP44* and *CNMD* as an undifferentiation marker for detecting undifferentiated cells present in a differentiated cell population.

The present invention also provides a kit for detecting undifferentiated cells, comprising a reagent capable of detecting the expression of at least one gene selected from the group consisting of *ESRG*, *VSNL1*, *THY1*, *SFRP2*, *SPP1*, *USP44* and *CNMD* and/or a reagent capable of measuring the promoter activity of the gene.

As the reagent capable of detecting the expression of the gene, primers, probes, antibodies or the like may be enumerated. Examples are: a set of oligonucleotide primers capable of specifically amplifying the transcription product (mRNA) or cDNA of at least one gene selected from the group consisting of *ESRG*, *VSNL1*, *THY1*, *SFRP2*, *SPP1*, *USP44* and *CNMD*; a nucleotide probe specifically hybridizing with the transcription product (mRNA) or cDNA of at least one gene selected from the group consisting of *ESRG*, *VSNL1*, *THY1*, *SFRP2*, *SPP1*, *USP44* and *CNMD*; and an antibody specifically binding to the protein (translation product) translated from the transcription product (mRNA) of at least one gene selected from the group consisting of *ESRG*, *VSNL1*, *THY1*, *SFRP2*, *SPP1*, *USP44* and *CNMD.* The set of oligonucleotide primers may be a set of primers capable of amplifying a target sequence (usually, approximately 50-180 bp) in the nucleotide sequence of the transcription product (mRNA) or cDNA of at least one gene selected from the group consisting of *ESRG, VSNL1*, *THY1*, *SFRP2*, *SPP1*, *USP44* and *CNMD.* Such a set of primers may be so designed that they have sequences complementary to both ends of the target sequence. The length of oligonucleotide primers may, for example, be 15-35 nucleotides, preferably 18-27 nucleotides. The nucleotide probe may be one hybridizing with the transcription product (mRNA) or cDNA of at least one gene selected from the group consisting of *ESRG*, *VSNL1*, *THY1*, *SFRP2*, *SPP1*, *USP44* and *CNMD* under stringent conditions. The nucleotide probe may be so designed that it has a part or whole of the nucleotide sequence of the above mRNA or cDNA, or a sequence complementary thereto. Stringent conditions may be appropriately selected. The length of nucleotide probes may be usually 1,000 nucleotides or less, preferably 100 nucleotides or less, more preferably 50 nucleotides or less, and still more preferably 14-30 nucleotides. The nucleotide probe may be either single-stranded or double-stranded. The antibody may be either monoclonal or polyclonal. As used herein, the term "antibody" is a concept encompassing not only full-length antibodies but also antibodies of smaller molecular sizes such as Fab, F(ab)'₂, ScFv, Diabody, V_{H}, V_{L}, Sc(Fv)₂, Bispecific sc(Fv)₂, Minibody, ScFv-Fc monomer and ScFv-Fc dimer. Probes and antibodies may be immobilized on a solid phase (such as substrate, beads, membrane, etc.).

The reagent of the present invention may be labeled. For example, primers may be labeled with a fluorescent substance, a quencher, or the like; and probes and antibodies may be labeled with a radioactive isotope, an enzyme, a luminescent substance, a fluorescent substance, biotin or the like. In the case where a target molecule (which, in the present invention, is a protein as the expression product of at least one gene selected from the group consisting of *ESRG*, *VSNL1*, *THY1*, *SFRP2*, *SPP1*, *USP44* and *CNMD*) is to be detected by first reacting a primary antibody that specifically binds to the target molecule and then reacting a secondary antibody that binds to the primary antibody, the secondary antibody may be labeled (the primary antibody is not labeled).

As the reagent capable of measuring the promoter activity of the gene, a gene sequence in which a reporter protein is ligated downstream of the promoter, a vector incorporating the gene sequence, or the like may be enumerated. Examples of the reporter protein include, but are not limited to, fluorescent proteins such as luciferase or GFP, and proteins such as CD antigen that are expressed in the cell membrane. As the vector, plasmid vectors are preferable.

The kit of the present invention may further comprise reagents for detecting genes with primers (e.g., DNA polymerase, buffer, magnesium ion, dNTPs, probe, etc.), reagents for detecting genes with probes (e.g., buffer, antibody, substrate, etc.), reagents for detecting genes with antibodies (e.g., secondary antibody, substrate, buffer, etc.), reagents for measuring the promoter activity of genes (e.g., buffer, luminescent substrate, antibody, etc.), instruments (reaction vessel, pipette, etc.), written instructions for using the kit, control samples, control data for analyzing measurement results, and so forth.

### EXAMPLES

Hereinbelow, the present invention will be described more specifically with reference to the following Examples. However, the scope of the present invention is not limited to these Examples.

### [Examples 1]

### Materials and Methods

### • iPSC

iPS cells kindly provided by Kyoto University and University of Tokyo were used (TkDA3-4, 1231A3, 1383D2, 1383D6 and Ff01).

### • Mesodermal Cell (Mesoderm) Differentiation

Undifferentiated iPS cells were seeded on laminin-coated dishes in the presence of ROCK inhibitor (Y-27632) at a density of 1-2 x 10⁴ cells/cm². Cells were cultured for 4 days in the presence of DMEMF12+B27+CHIR+BMP4 to obtain mesodermal cells (Mesoderm).

### • Ectodermal Cell (Ectoderm) Differentiation

Undifferentiated iPS cells were seeded on laminin-coated dishes in the presence of ROCK inhibitor (Y-27632) at a density of 1 x 10⁵ cells/cm². Cells were cultured for 7 days in the presence of DMEM F12+KSR+2-ME+EGF+2 bFGF+SB431542+BIO to obtain ectodermal cells (Ectoderm). Alternatively, ectodermal cells (Ectoderm) may also be obtained by culturing in the presence of KBM-NSC+B27+bFGF+ hLIF+CHIR99021+SB431542 for 7 days.

### • Residual Undifferentiated Cells

In order to quantify undifferentiated cells remaining in the cells differentiated from iPSC, the present inventors applied the method described by Tano et al. Briefly, differentiated cells were detached with trypsin, seeded in ROCK inhibitor-containing StemFit in 24-well plates at 1.6 x 10⁵ cells/well, and cultured at 37°C with the culture medium being exchanged with StemFit every day. One week later, immunostaining was performed and positive colonies were counted.

### • Colony Counting

Immunostained samples were photographed with a microscope. Colonies on those photographs were counted visually. The number of residual undifferentiated iPS cells was based on the assumption that one colony of undifferentiated iPSC was derived from one undifferentiated iPS cell.

### • Hepatocyte Differentiation

Undifferentiated iPS cells were seeded on laminin-coated dishes in the presence of ROCK inhibitor (Y-27632) at a density of 5-10 x 10⁴ cells/cm². Cells were cultured for 6 days in the presence of RPMI+B27+activin A+Wnt3A to obtain definitive endoderm cells (DE). The resultant cells were further cultured for 4 days in the presence of KO-DMEM+KSR+DMSO+2-Mercaptoethanol to obtain hepatic endoderm cells (HE).

### • Immunostaining

In order to detect undifferentiated cells, the present inventors performed immunostaining using primary antibodies against pluripotency markers SOX2 and TRA-1-60 (Cell Signaling Technologies) and the corresponding secondary antibodies (Thermo Fisher Scientifics). Cells were fixed with 4% paraformaldehyde for 15 min, and washed twice with PBS. Cell membranes were permeabilized with 0.1% TritonX-100 in PBS (PBST) for 10 min and subsequently blocked with 5% FBS in PBST. After one hour, blocking buffer was removed. Appropriately diluted solution of the primary antibodies was added and cells were treated at 4°C overnight. Then, cells were washed three times with PBS. Diluted solution of the secondary antibodies was applied and the mixture was allowed to settle undisturbed for one hour at room temperature under light shielding conditions. In the last step, cells were washed three times with PBS, followed by addition of Apathy's Mounting Media (FUJIFILM Wako Pure Chemical Corporation) for observation.

### • Microscope (KEYENCE, etc.)

Whole-well images were obtained using an all-in-one fluorescence microscope BZ-X710 in bright field and blue, green and red fluorescence channels with objective lenses x4 and ×10.

### • qPCR

RNA was extracted from cells using PureLink RNA Mini Kit (Thermo Fisher) and cDNA was synthesized by reverse transcription using High-Capacity cDNA Reverse Transcription Kit (Thermo Fisher). Then, qPCR was performed using the following primers and Universal Probe Library (Roche).

### ESRG

Forward: tgggatggagccatagaagt (SEQ ID NO: 1)
Reverse: tgggtctttcaagaagttcctc (SEQ ID NO: 2)
Probe: #52

### VSNL1

Forward: gaactgttgagttttatcattttcgt (SEQ ID NO: 3)
Reverse: caggggccagtttgctatt (SEQ ID NO: 4)
Probe: #60

### THY1

Forward: cagaacgtcacagtgctcaga (SEQ ID NO: 5)
Reverse: gaggagggagagggagagc (SEQ ID NO: 6)
Probe: #66

### SFRP2

Forward: gctagcagcgaccacctc (SEQ ID NO: 7)
Reverse: tttttgcaggcttcacatacc (SEQ ID NO: 8)
Probe: #83

### SPP1

Forward: gagggcttggttgtcagc (SEQ ID NO: 9)
Reverse: caattctcatggtagtgagttttcc (SEQ ID NO: 10)
Probe: #18

### USP44

Forward: ccctcagaccagaatgcttta (SEQ ID NO: 11)
Reverse: cattgcagtgtacccagaacc (SEQ ID NO: 12)
Probe: #9

### LECT1 (CNMD)

Forward: actcacaagccttcaatcctg (SEQ ID NO: 13)
Reverse: tctagggtcgaatgtcatgct (SEQ ID NO: 14)
Probe: #18

### • Spike-In Experiments

iPS cells cultured under conditions for maintaining undifferentiated state were caused to become included in cells resulting from directed differentiation at the proportions indicated in the Figures. Using the resultant cell mixtures, a test for residual undifferentiated cells, qPCR, etc. were performed.

### • Statistics

P values <0.05 were considered significant by Student's t-test. Correlation coefficients were calculated based on Pearson product-moment correlation coefficient.

### Results

### ▪ LIN28 is not useful as an indicator for undifferentiated iPSC in the liver.

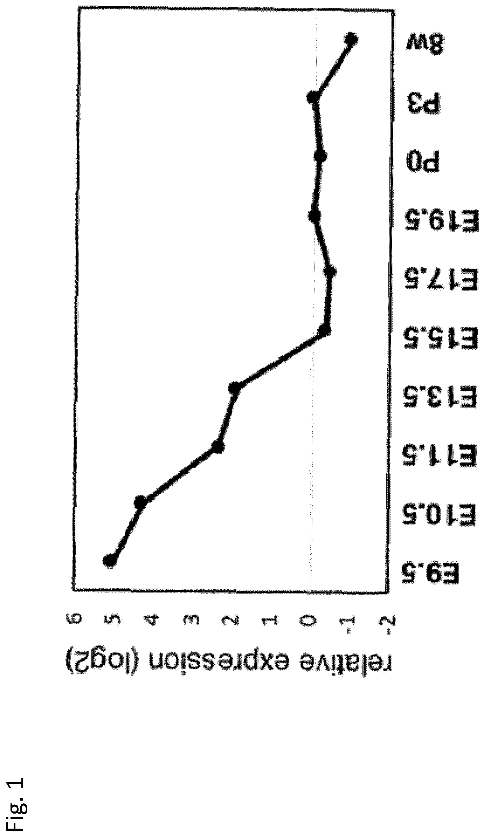

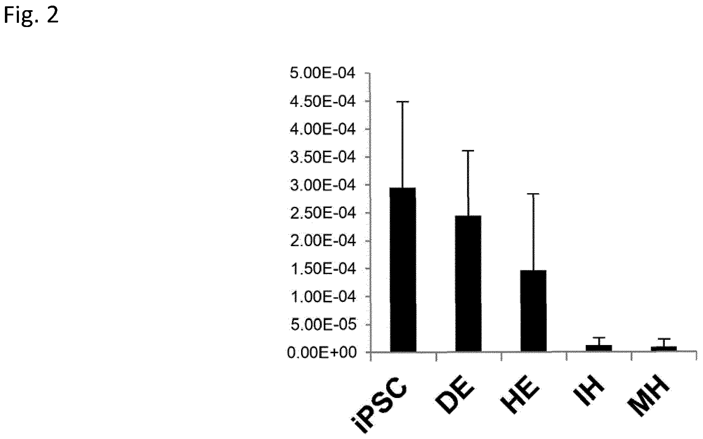

It has been reported that *LIN28* (*LIN28A*) is useful as an indicator for residual undifferentiated iPSC in retinal pigment epithelial cells (RPE) resulting from directed differentiation of iPSC (Kuroda T. et al., PLoS ONE 7(5):e37342.(2012)). When *LIN28* expression was observed in mouse liver during development, it became clear that *LIN28* expression was high until E13.5 compared to the expression in adult (8w) (see Fig. 1). It also became clear that in iPS cell-derived endodermal cells, i.e., definitive endoderm cells (DE) and hepatic endoderm cells (HE), *LIN28* expression hardly decreased as compared to the expression in undifferentiated iPS cells (see Fig. 2).

### ▪ Extraction of genes that are specifically expressed in iPS cells with high yield

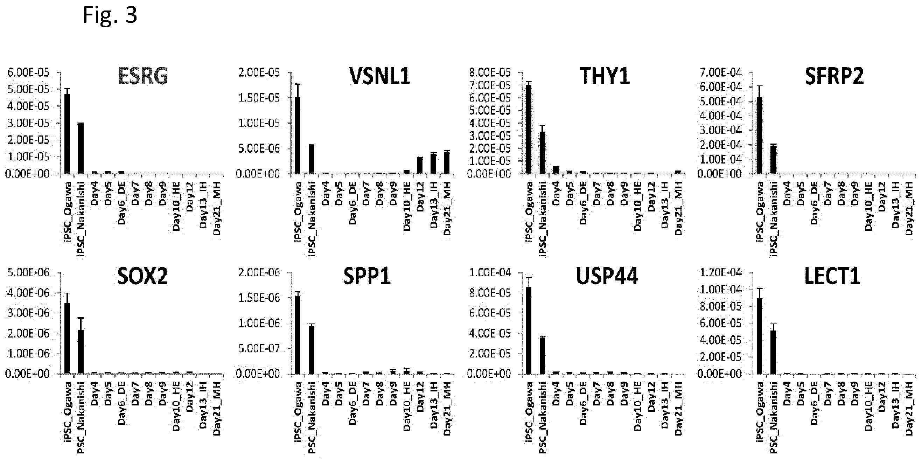

In order to extract marker genes as indicators for residual undifferentiated iPSC that might be useful in iPS cell-derived hepatic cells, the present inventors performed microarray analyses of mouse developmental stages and single cell RNA sequence analyses during processes of directed differentiation of iPS cell-derived hepatic cells, whereby a plurality of genes were extracted that were expressed in undifferentiated iPS cells both specifically and with high yield but whose expression was low in differentiated cells. By subjecting the microarray-extracted genes to qPCR, genes were extracted that were expressed in iPS cells with high yield but whose expression decreased in differentiated cells (Fig. 3).

### ▪ Quantification of residual undifferentiated cells by formation of undifferentiated cell colonies

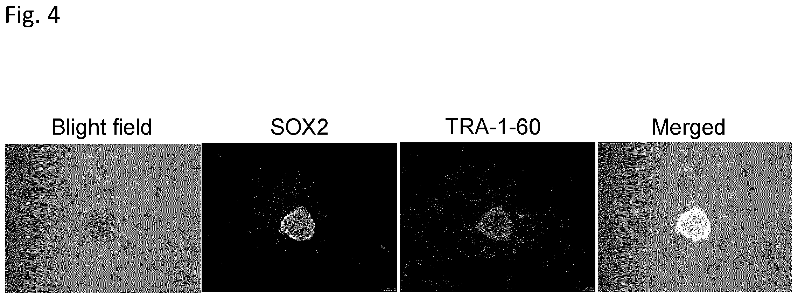

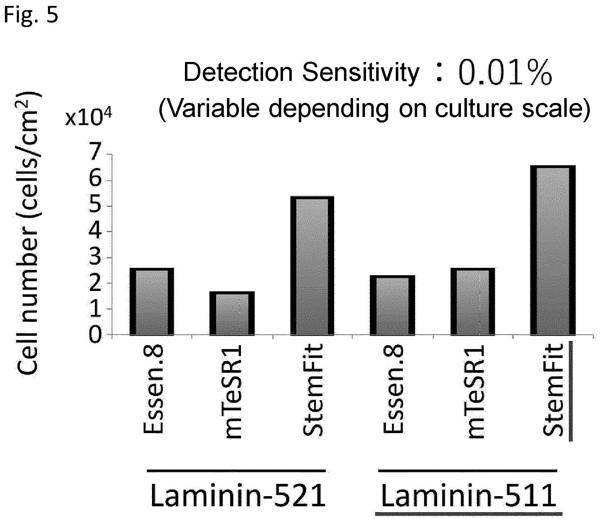

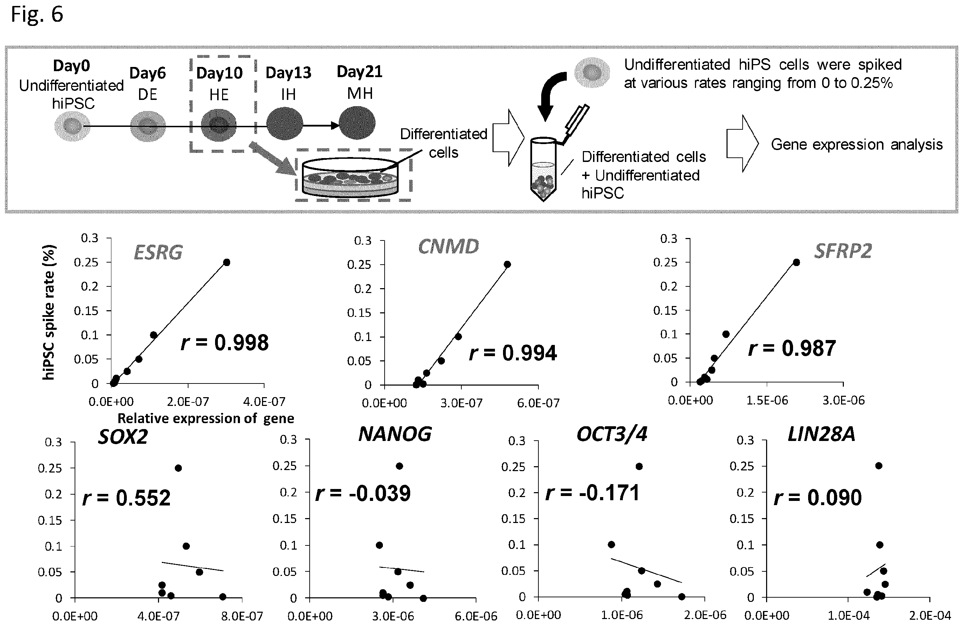

In order to examine whether the expression of the marker genes selected in Fig. 3 correlates with the actual number of residual undifferentiated iPS cells, the present inventors first optimized a technique for evaluating the number of residual undifferentiated iPS cells (Figs. 4 and 5) based on the previously reported technique (Tano K et al., PLoS One. 2014; 9(10):e110496). Briefly, using iPS cell-derived hepatic progenitor cells (HE) resulting from directed differentiation, residual undifferentiated cell tests were carried out under conditions optimized in Figs. 4 and 5 to calculate the number of residual undifferentiated cells, which was then analyzed for correlation with the marker gene expression as determined by qPCR (Fig. 6). The expression levels of novel markers for residual undifferentiated hiPSC exhibited high correlation with the inclusion ratio of undifferentiated hiPSC.

### ▪ Examination of detection limits of respective marker genes (undifferentiated iPSC spike-in experiments)

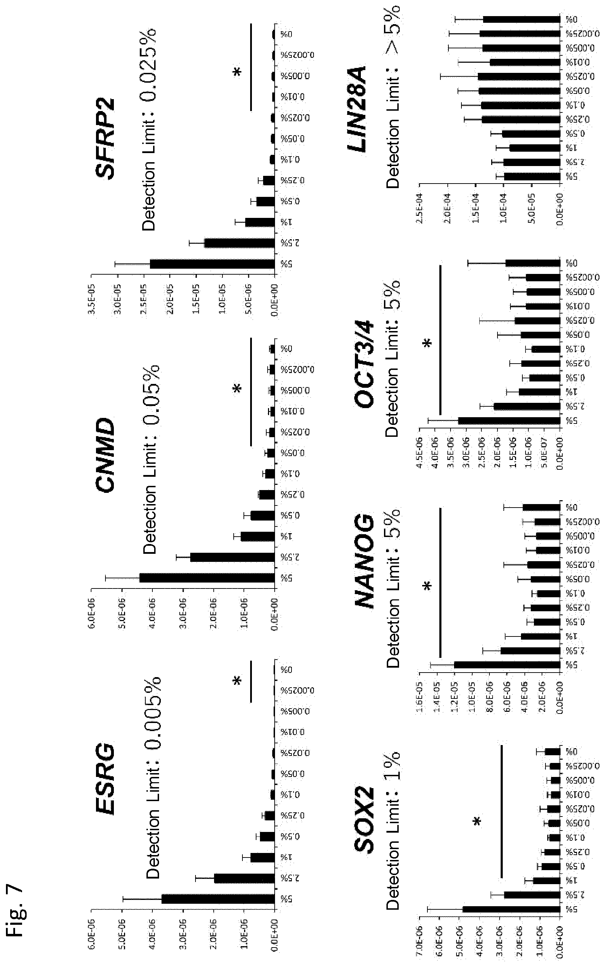

iPS cells cultured under conditions for maintaining undifferentiated state were caused to become included in iPS-cell derived hepatic progenitor cells (HE) resulting from directed differentiation at the proportions indicated in the Figures, and qPCR was performed on the resultant cell mixtures (Fig. 7). As a result, detection sensitivity of 1000-fold or more was achieved as compared to the conventional method using *LIN28A.*

### ▪ Residual undifferentiated cell experiments in differentiated mesodermal and ectodermal cells

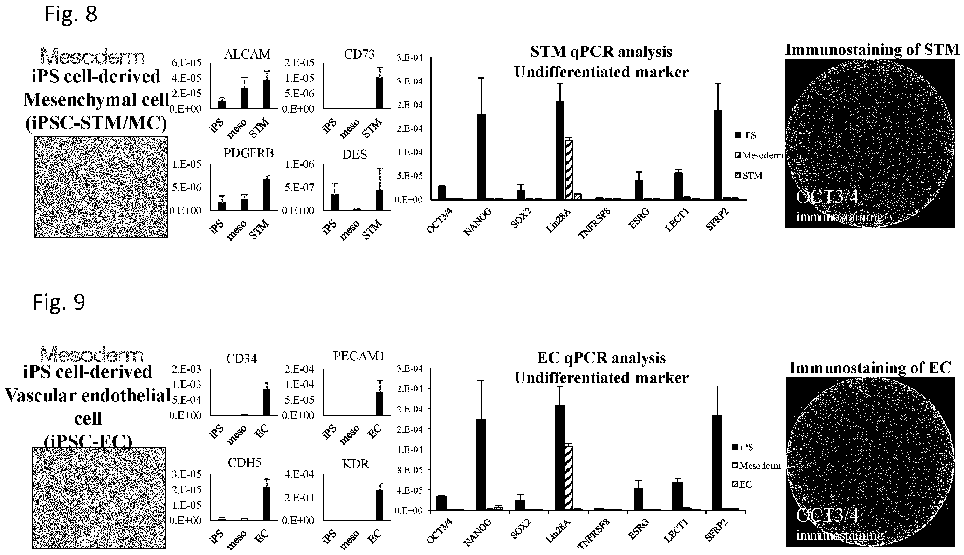

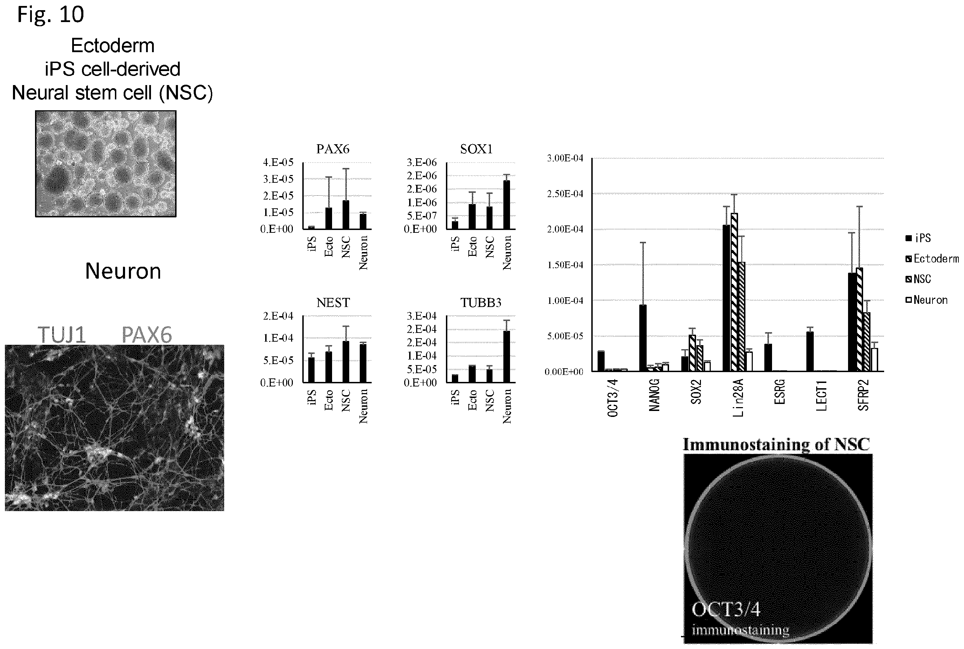

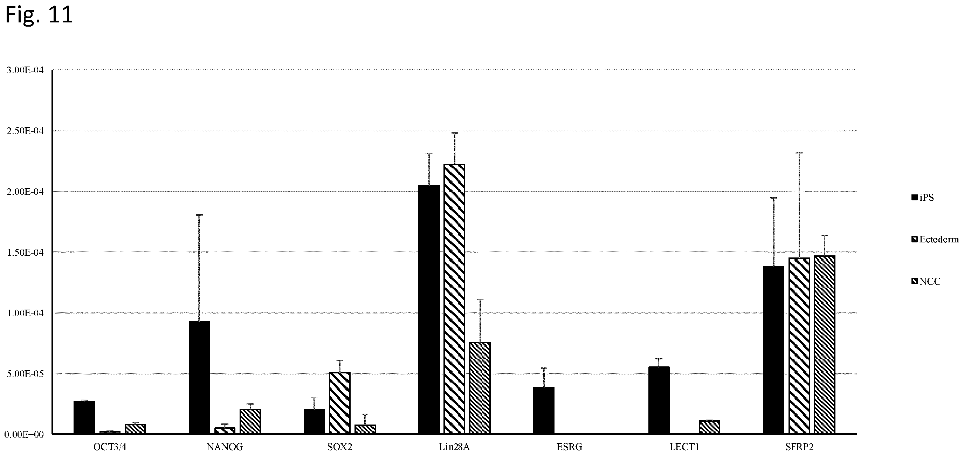

As mesoderm-derived cells, iPS cell-derived *septum transversum* mesenchyme cells (iPSC-STM) and vascular endothelial cells (iPSC-EC) both resulting from directed differentiation were used. Residual undifferentiated iPSC experiments and qPCR-based examination of marker gene expression were performed with these cells. The results revealed that in both iPSC-STM and iPSC-EC, expression of *ESRG, SFRP2* and *CNMD* decreased in differentiated cells including no residual undifferentiated cells, which suggested the usefulness of these three genes as markers for residual undifferentiation (Figs. 8 and 9). As ectoderm-derived cells, iPS cell-derived neural stem cells (iPSC-NSC) and neural cells (Neuron) both resulting from directed differentiation were used. Residual undifferentiated iPSC experiments and qPCR-based examination of marker gene expression were performed with these cells. The results revealed that in both iPSC-NSC and Neuron, expression *of ESRG* and *CNMD* decreased in differentiated cells including no residual undifferentiated cells, which suggested the usefulness of these genes as markers for residual undifferentiation (Fig. 10). On the other hand, the expression of *LIN28A* did not decrease, so it became clear that in iPS cell-derived neural stem cells and neural cells (Neuron), too, there was no correlation between residual undifferentiated cells and *LIN28A* expression. Similar results were also obtained in qPCR-based measurement in iPSC-derived neural crest cells resulting from directed differentiation (NCC) (Fig. 11).

### ▪ Residual undifferentiated cell experiments in differentiated cells prepared with STEMdiff™ Trilineage Differentiation Kit

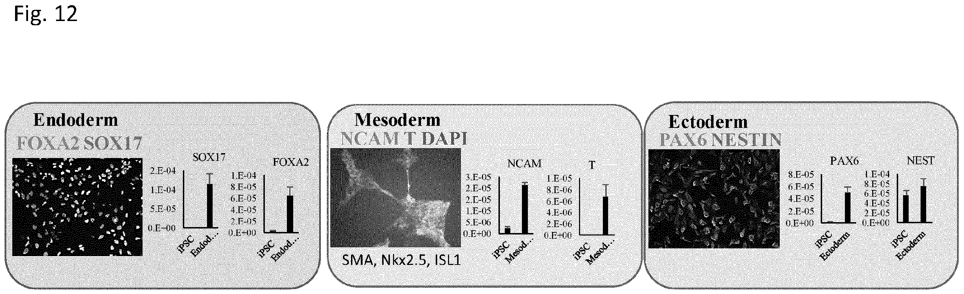

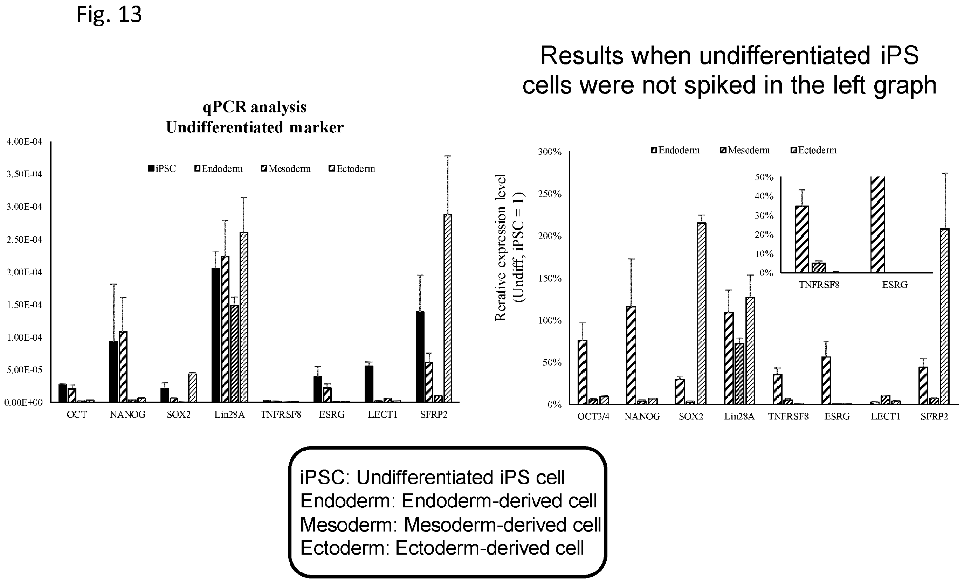

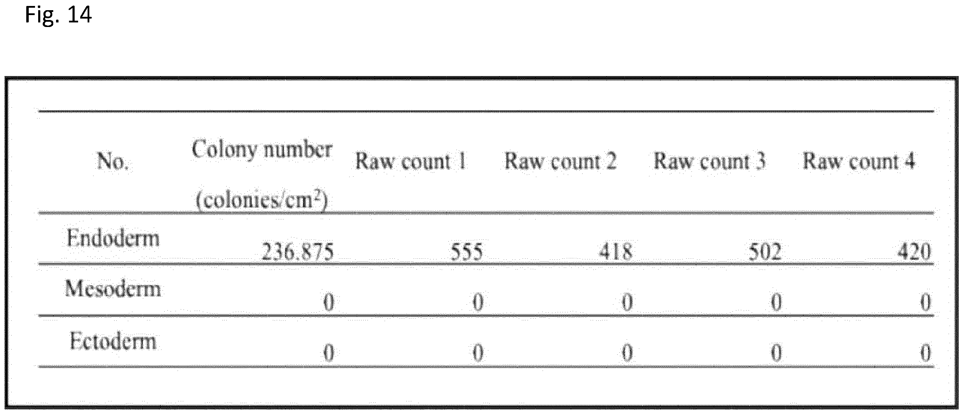

Further, in order to show that the above genes are generally useful as markers for residual undifferentiated cells in iPS cell-derived cells resulting from directed differentiation, the present inventors performed examination using cells that resulted from directed differentiation with a commercial directed differentiation kit (STEMdiff™ Trilineage Differentiation Kit; STEMCELL Technologies) (Fig. 12). As a result, residual undifferentiated cells were observed in endoderm-derived cells resulting from directed differentiation of iPS cells but at the same time, marker gene expression was high in correlation with the number of residual undifferentiated cells. Therefore, correlation between the number of residual undifferentiated cells and marker expression was observed in any of the cell lineages resulting from directed differentiation of iPS cells, i.e., endoderm-derived cells, mesoderm-derived cells and ectoderm-derived cells (Figs. 13 and 14).

### Discussion

Detection and exclusion of undifferentiated cell contamination in iPS (ES) cell-derived differentiated cells which contribute to applications in regenerative medicine are two important issues in ensuring the safety of all products processed from iPS (ES) cell-derived cells. To date, rapid evaluation of undifferentiated cell contamination by verification of *LIN28A* expression in retinal pigment epithelial cells (RPE) has been reported. However, *LIN28A* is expressed in the liver during mouse development. As a matter of fact, expression of *LIN28A* is also observed in cells resulting from directed differentiation of human iPS cells, and it became clear that this expression did not correlate with the presence or absence of undifferentiated cells actually remaining in differentiated cells. Accordingly, the present inventors extracted a plurality of markers for residual undifferentiated cells in iPS cell-derived hepatic cells. It has been revealed that these markers are useful as indicators of residual undifferentiated cells not only in hepatic cells which are endoderm-derived cells, but also in mesodermal cells, mesoderm-derived septum transversum mesenchyme cells, mesenchymal cells and vascular endothelial cells, all of which result from directed differentiation of iPS cells. Further, it has become clear that markers whose expression correlates with residual undifferentiated cells are also contained in ectodermal cells, ectoderm-derived neural stem cells, neural crest cells and neural cells, all of which result from directed differentiation of iPS cells. On the other hand, the expression of *LIN28A* did not decrease, so it was revealed that in iPS cell-derived neural stem cells, too, *LIN28A* does not serve as an indicator for residual undifferentiated cells. In view of these results, the technique for detecting residual undifferentiated iPS cells using the plurality of marker genes as extracted in accordance with the present invention is expected to provide a simple and quick tool for ensuring the safety of various products processed from iPS (ES) cell-derived cells.

References:
▪ Kuroda T. et al., PLoS ONE 7(5):e37342 (2012)
▪ Tano et al., PLoS One. 2014 27;9(10):e110496

### [Example 2]

Without directly detecting the expression of the marker gene of the present invention, residual undifferentiated cells can be detected if a reporter protein gene (e.g., fluorescent protein genes such as luciferase gene and GFP gene or genes such as *mouse CD4* that are expressed on cell surfaces and whose expression can be detected specifically as with antibody) is incorporated into the marker gene of the present invention under the control of the promoter region associated with regulation of its expression.

### [Example 3]

It becomes possible to select a highly safe undifferentiated cell clone from a plurality of iPS cell clones. Briefly, the expression of the marker gene of the present invention is evaluated in cells of interest as differentiated from the clones and cells that result from directed differentiation of the clones with a commercial kit such as a tri-lineage differentiation kit, and then those cell clones which are characterized by decreased expression of the marker gene are selected.

### [Example 4]

Differentiated cells are transplanted into a model animal over a long period of time, and engrafting cells are collected. By evaluating the expression of the marker gene of the present invention by qPCR, the method described in Example 2 above or otherwise, undifferentiated cells in the formed tissue are detected.

All publications, patents and patent applications cited herein are incorporated herein by reference in their entirety.

### INDUSTRIAL APPLICABILITY

The present invention is applicable to detection and evaluation of the undifferentiated cells that remain or become included in differentiated cells for use in regenerative medicine.

### SEQUENCE LISTING FREE TEXT

### <SEQ ID NOS: 1 to 14>

DNA sequences of primers are shown.

## Claims

1. A method of detecting undifferentiated cells, comprising measuring the expression level and/or the promoter activity of at least one gene selected from the group consisting of *ESRG*, *VSNL1*, *THY1*, *SFRP2*, *SPP1*, *USP44* and *CNMD* in a differentiated cell population.

2. The method of claim 1, wherein the undifferentiated cell is embryonal carcinoma cell (EC cell), embryonic stem cell (ES cell), induced pluripotent stem cell (iPS cell) or embryonic germ cell (EG cell), and the differentiated cell population is a population of cells which have been differentiated from the undifferentiated cell.

3. The method of claim 1 or 2, wherein the differentiated cell population is a population of differentiated endodermal, mesodermal or ectodermal cells.

4. The method of claim 3, wherein the differentiated endodermal cells are hepatic endoderm cells.

5. The method of claim 3, wherein the differentiated mesodermal cells are *septum transversum* mesenchyme cells, mesenchymal cells or vascular endothelial cells.

6. The method of claim 3, wherein the differentiated ectodermal cells are neural stem cells, neural crest cells or neural cells.

7. The method of any one of claims 1 to 6, wherein the expression level of the gene is measured as the amount of mRNA or protein.

8. The method of any one of claims 1 to 7, wherein the expression level of the gene is measured by qPCR, digital PCR, immunostaining, *in situ* hybridization, RNA sequencing, microarray, NanoString, antibody array, flow cytometry or mass spectrometry.

9. A method of selecting a highly safe undifferentiated cell clone, comprising measuring the expression level and/or the promoter activity of at least one gene selected from the group consisting of *ESRG*, *VSNL1*, *THY1*, *SFRP2*, *SPP1*, *USP44* and *CNMD* in differentiated endodermal, mesodermal or ectodermal cells that result from directed differentiation of an undifferentiated cell clone.

10. The method of claim 9, wherein the undifferentiated cell clone is an embryonal carcinoma cell (EC cell) clone, an embryonic stem cell (ES cell) clone, an induced pluripotent stem cell (iPS cell or iPSC) clone or an embryonic germ cell (EG cell) clone.

11. The method of claim 10, wherein the undifferentiated cell clone is an iPS cell clone.

12. A method of detecting undifferentiated cells, comprising measuring the expression level and/or the promoter activity of at least one gene selected from the group consisting of *ESRG*, *VSNL1*, *THY1*, *SFRP2*, *SPP1*, *USP44* and *CNMD* in a tissue formed by transplanting differentiated cells into a model animal.

13. A method of using at least one gene selected from the group consisting of *ESRG*, *VSNL1*, *THY1*, *SFRP2*, *SPP1*, *USP44* and *CNMD* as an undifferentiation marker for detecting undifferentiated cells in a differentiated cell population.

14. A kit for detecting undifferentiated cells, comprising a reagent capable of detecting the expression of at least one gene selected from the group consisting of *ESRG*, *VSNL1*, *THY1*, *SFRP2*, *SPP1*, *USP44* and *CNMD* and/or a reagent capable of measuring the promoter activity of the gene.

15. The kit of claim 14, wherein the reagent capable of detecting the expression of the gene is primers, probes or antibodies.

16. The kit of claim 14, wherein the reagent capable of measuring the promoter activity of the gene is a gene sequence in which a reporter protein is ligated downstream of the promoter or a vector incorporating the gene sequence.


SEQUENCE LISTING

<110>  Pubilic University Corporation Yokohama City University

<120>  A method for detecting undifferentiated cells

<130>  FP-263PCT

<150>  JP2018-115025
<151>  2018-06-15

<160>  14

<170>  PatentIn version 3.5

<210>  1
<211>  20
<212>  DNA
<213>  Artificial Sequence

<220>
<223>  primer

<400>  1
tgggatggag ccatagaagt                                                   20


<210>  2
<211>  22
<212>  DNA
<213>  Artificial Sequence

<220>
<223>  primer

<400>  2
tgggtctttc aagaagttcc tc                                                22


<210>  3
<211>  26
<212>  DNA
<213>  Artificial Sequence

<220>
<223>  primer

<400>  3
gaactgttga gttttatcat tttcgt                                            26


<210>  4
<211>  19
<212>  DNA
<213>  Artificial Sequence

<220>
<223>  primer

<400>  4
caggggccag tttgctatt                                                    19

<210>  5
<211>  21
<212>  DNA
<213>  Artificial Sequence

<220>
<223>  primer

<400>  5
cagaacgtca cagtgctcag a                                                21


<210>  6
<211>  19
<212>  DNA
<213>  Artificial Sequence

<220>
<223>  primer

<400>  6
gaggagggag agggagagc                                                   19


<210>  7
<211>  18
<212>  DNA
<213>  Artificial Sequence

<220>
<223>  primer

<400>  7
gctagcagcg accacctc                                                    18


<210>  8
<211>  21
<212>  DNA
<213>  Artificial Sequence

<220>
<223>  primer

<400>  8
tttttgcagg cttcacatac c                                                21


<210>  9
<211>  18
<212>  DNA
<213>  Artificial Sequence

<220>
<223>  primer

<400>  9
gagggcttgg ttgtcagc                                                    18


<210>  10
<211>  25
<212>  DNA

<213>  Artificial Sequence

<220>
<223>  primer

<400>  10
caattctcat ggtagtgagt tttcc                                             25


<210>  11
<211>  21
<212>  DNA
<213>  Artificial Sequence

<220>
<223>  primer

<400>  11
ccctcagacc agaatgcttt a                                                 21


<210>  12
<211>  21
<212>  DNA
<213>  Artificial Sequence

<220>
<223>  primer

<400>  12
cattgcagtg tacccagaac c                                                 21


<210>  13
<211>  21
<212>  DNA
<213>  Artificial Sequence

<220>
<223>  primer

<400>  13
actcacaagc cttcaatcct g                                                 21


<210>  14
<211>  21
<212>  DNA
<213>  Artificial Sequence

<220>
<223>  primer

<400>  14
tctagggtcg aatgtcatgc t                                                 21